# EUROPEAN PATENT APPLICATION

(11) **EP 2 220 997 A1**
(43) Date of publication of application: **25.08.2010**
(21) Application number: 09153497.4
(22) Date of filing: 24.02.2009
(51) Int. Cl.: A61B 5/103, A61B 5/11

(54) **Device, system and method for monitoring motion sequences**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

A device (1) for monitoring motion sequences of a person, comprises a flexible tube (2) with a proximal and a distal end, the tube being adapted to receive a limb of a person; a loop (3) disposed at the proximal end of the tube (2), the loop (3) being adapted to be worn around a body part of the person; and at least one sensor (4) connected to the tube (2), the sensor (4) being selected from the group comprising motion sensors and/or physiological parameter sensors. A system (10) for monitoring motion sequences of a person, comprises such a device (1) and a data processing unit (11). A sensor (4) of the device (1) is adapted to transmit sensor data to the data processing unit (11) and the data processing unit (11) is adapted to perform calculations on the received sensor data. In a method, such a system (10) is provided and sensor data is compared to stored data.

## Description

### FIELD OF THE INVENTION

The invention relates to the field of monitoring motion sequences of a moveable part of the body. In particular, the invention relates to a device for monitoring motion sequences of a person, a system comprising such a device and method for monitoring motion sequences of a person using such a system.

### BACKGROUND OF THE INVENTION

A growing market has developed for tools and systems that track human and other bodies at rest and in motion. The applications for such systems vary considerably, and include such areas as the creation of computer-generated and graphical animations, the analysis of human-computer interactions, the assessment of performance athletics and other biomechanical activities, and the evaluation of workplace and related activities for general ergonomical fitness or, finally, physiotherapy.

At the moment, physiotherapy is very labor intensive as a physiotherapist can treat only one patient at a time. This makes physiotherapy very expensive, resulting in only a limited number of reimbursed treatments for the patient. The outcome for the patient would be improved if he could do more or longer physiotherapy exercises, but the costs of the physiotherapist being present all the time prevents this. One group of patients that would benefit particularly from more supervised exercises are those suffering from a hemiplegic stroke.

Sensors may be placed in garments to measure physiological parameters of the user and to employ the generated data for further use. Typically, such garments are used in sports and medical environments to measure the performance of the user.

Such an arrangement is mentioned in US 6,050,962, which discloses a sensing system provided for measuring various joints of a human body for applications for performance animation, biomechanical studies and general motion capture. One sensing device of the system is a linkage-based sensing structure comprising rigid links interconnected by revolute joints, where each joint angle is measured by a resistive bend sensor or other convenient goniometer. Such a linkage-based sensing structure is typically used for measuring joints of the body, such as the shoulders, hips, neck, back and forearm, which have more than a single rotary degree of freedom of movement. In one embodiment of the linkage-based sensing structure, a single long resistive bend sensor measures the angle of more that one revolute joint. The terminal ends of the linkage-based sensing structure are secured to the body such that movement of the joint is measured by the device.

A second sensing device of the sensing system comprises a flat, flexible resistive bend sensor guided by a channel on an elastic garment. Such a flat sensing device is typically used to measure various other joints of the body which have primarily one degree of freedom of movement, such as the elbows, knees and ankles. Combining the two sensing devices as described, the sensing system has low sensor bulk at body extremities, yet accurately measures the multi-degree-of freedom joints nearer the torso. Such a system can operate totally untethered, in real time, and without concern for electromagnetic interference or sensor occlusion.

However, there is still a significant drawback in the reference as described above, particularly in connection with the resistive bend sensor guided by a channel on an elastic garment. Patients suffering from the effects of a hemiplegic stroke or who are otherwise impaired with their arm movements may not be capable of fitting this elastic garment onto their arm or leg in order to conduct rehabilitation exercises. Thus, a second person is needed to assist them.

### SUMMARY OF THE INVENTION

Accordingly, the present invention is directed towards a device for monitoring motion sequences of a person, comprising a flexible tube with a proximal and a distal end, the tube being adapted to receive a limb of a person, further comprising a loop disposed at the proximal end of the tube, the loop being adapted to be worn around a body part of the person and further comprising at least one sensor connected to the tube, the sensor being selected from the group comprising motion sensors and/or physiological parameter sensors.

Another aspect of the present invention is a system for monitoring motion sequences of a person, comprising a device according to the invention and a data processing unit, wherein a sensor of the device is adapted to transmit sensor data to the data processing unit and the data processing unit is adapted to perform calculations on the received sensor data.

Another aspect of the present invention is a method for monitoring motion sequences of a person, comprising the steps of:
a) providing a system according to the invention;
b) transmitting data from a motion sensor to the data processing unit; and
c) comparing the data to stored data representing exercise motions.

Another aspect of the present invention is the use of a device according to the invention for monitoring motion sequences of a person.

The present invention has the general advantage that it is easier for a single person with an impaired limb to perform exercises for rehabilitation while being alone. In particular, the device according to the invention can be put on without further assistance. For example, in the case of an impaired arm, the device is worn like a sleeve. This can be achieved even if the impaired arm cannot be controlled very well by the person. The loop on the device prevents the device from slipping off the limb, thus securing the device on the body. Additionally, the loop that is worn around a body part helps to prevent a rotation of the device on the limb. This keeps motion sensors in place.

### BRIEF DESCRIPTION OF THE DRAWINGS

- FIG. 1: shows a device according to the invention worn on the arm of a person
- FIG. 2: shows a device according to the invention worn on the leg of a person
- FIG. 3: shows the control unit 5 and the person
- FIG. 4: shows how a device according to the invention is worn as a sequence of steps

### DETAILED DESCRIPTION OF EMBODIMENTS

Before the invention is described in detail, it is to be understood that this invention is not limited to the particular component parts of the device described or steps of the methods described as such device and method may vary. It is also to be understood that the terminology used herein is for purpose of describing particular embodiments only, and is not intended to be limiting. It must be noted that, as used in the specification and the appended claim, the singular forms "a", "an" and "the" include singular and/or plural referents unless the context clearly dictates otherwise. Thus, for example, reference to "a sensor" or "the sensor" may include several sensors, and the like. Furthermore, the word "comprising" does not exclude other elements or steps.

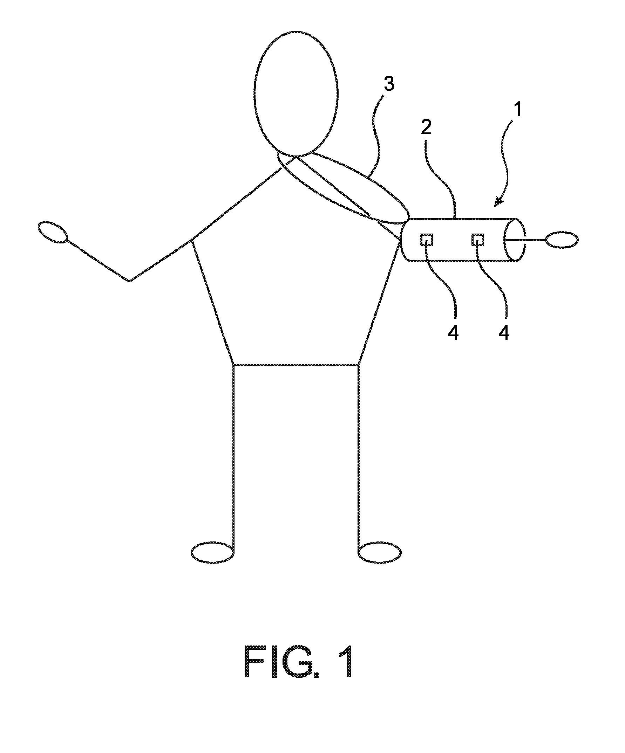

The device according to the present invention will now be described in general and with additional reference to FIG. 1. A person is schematically shown which is wearing a device 1 according to the invention with a tube 2, a loop 3 and sensors 4 on his arm.

A flexible tube 2 as used in the context of the present invention is not only to be understood as being cylinder-shaped in the exact geometrical sense with a circular cross-section, but the cross-section may also be elliptical or have other, more irregular shapes. Being a tube, it is hollow and open at least at one longitudinal end. The tube 2 is flexible (as opposed to rigid), meaning that it can change its form, for example when it worn on a limb or when this limb is flexed.

The tube 2 is furthermore adapted to receive a limb of a person. This means, especially with respect to shape and size, that the tube 2 can be worn on a limb of a person. Examples for such limbs are a hand, a part of the hand, a forearm, a part of the forearm, an upper arm, a part of the upper arm, an arm, a torso, a part of the torso, a foot, a part of the foot, a calf, a part of the calf, a leg and/or combinations of the aforementioned limbs, when anatomically possible. Illustrative examples for the tube 2 would be in the form of a sleeve in order to receive an arm, as shown in FIG. 1, or in the form of a trouser leg in order to receive a leg.

It is within the scope of the invention that the tube 2 comprises outwardly extended sections to receive parts of the respective limb. Outwardly extended sections of the tube 2 may, for example, receive the elbow or the knee. This has two advantages. Firstly, the wearing comfort around the joint is increased. This shaping of the tube also prevents persons from wearing the tube 2 wrongly, for example in a twisted fashion on the arm. If the person is guided to wear the tube the same way every time, the sensor position on the limb will not vary as much. This is especially beneficial for sensors relating to the function of a muscle, such as electromyographical sensors. In order to give meaningful signals, the sensor needs to be placed on the intended muscle and not on another one.

When the tube 2 receives the limb of a person, by definition a proximal end and a distal end of the tube 2 are created. In accordance with the anatomical terminology, the proximal end is the end of the tube 2 which is closer to the body and the distal end is the end which is further away from the body. In the example of FIG. 1, the proximal end of tube 2 is the end at the shoulder of the person, the distal end is the end at the hand of the person.

With respect to the material of the tube 2, in principle there are no restrictions as long as it is flexible as outlined above. For example, the material of the tube may be a woven fabric, an elastic woven fabric, a knitted fabric, a polymer tube, or the like.

Furthermore, a loop 3 is disposed at the proximal end of the tube 2. In the example of FIG. 1, it is disposed at the end of the tube 2 near the shoulder of the person. It is preferred that the loop 3 is disposed as far as possible towards the proximal end. This reduces potential chafing of the loop 2 against the body of the person. With respect to its size and shape, the loop 3 is adapted to be worn around a body part of a person. By this, the tube 2 is fastened to the body. As seen in FIG. 1, the loop 3 may be worn around the neck. For drawing purposes, the loop 3 is shown here to be loosely fitting. However, in practice the loop will be tight-fitting. The loop 3 may be a closed loop with a fixed circumference. The loop 3 may also be of the type which can be opened and closed, for example by buttoning, by hook and loop fasteners or by buckling. Furthermore, the circumference of the loop 3 may be variable, for example by using a strap or ratchet mechanism. This allows to have a large loop 3 for pulling over the head, for example, and then to tighten it for wearing. It is also possible that the loop is elastic, for example by comprising an elastic material or an elastic woven fabric.

The loop 3 may be of the type where two ends of a strap are affixed to different places of the tube 2. The two ends of the strap may also be affixed to the same place of the tube 2. The latter case is shown in the figures.

The device 1 according to the invention further comprises at least one sensor 4 connected to the tube 2, wherein the sensor is selected from the group comprising motion sensor and/or physiological parameter sensors. Hence, the device 1 may display motion sensors, physiological sensors or a combination of motion sensors and physiological parameter sensors. The motion sensors serve to collect data representative of the motion of the person executing an exercise. In simple cases, the motion sensor 4 may be fastened to the tube 2. Physiological parameter sensors monitor the physical and physiological state of the person. This allows to better evaluate the well-being of the person conducting the exercises. The aforementioned sensors may also be integrated into the material of the tube, for example by weaving or knitting. It is furthermore possible that at least one sensor is additionally fastenable to the respective limb of the person by adjustable straps.

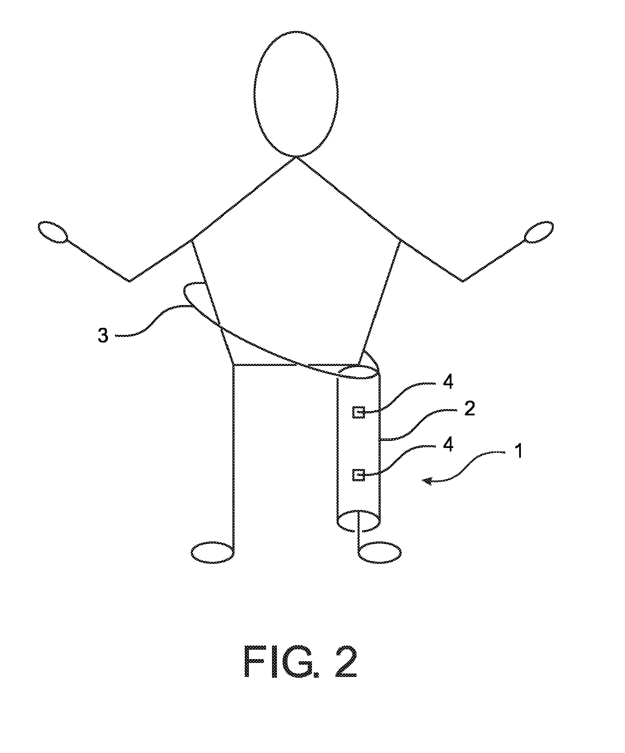

In order to give another example, FIG. 2 shows the device 1 according to the invention which is worn on the leg of the person. Here, the tube 2 receives the leg of the person and the loop 3 is worn around the waist.

In an embodiment of the device, the motion sensors are selected from the group comprising acceleration sensors, inertia sensors, gravity sensors, magnetometers, gyroscopes, optical tracking devices, tilt meters, distance meters, position meters, goniometers, and/or angle meters. Hence, these sensor types may be present alone or as a combination of the aforementioned sensors. Acceleration sensors, inertial sensors, gravity sensors, magnetometers and gyroscopes may also be combined into a highly integrated solid state sensor. This has the advantage of using very little space on the person's body. Such integrated sensors are commercially available, for example from PHILIPS under the name π-NODE. Optical tracking devices may be cameras that record the surroundings in order to interpret a movement. They also may be suitable marks to be tracked by cameras to determine the spatial position. Examples for distance meters include optical, acoustical, sonar, radar, capacitive and inductive distance meters. Angle meters may require a strap around the respective joint, such as the elbow, in order to fasten this type of motion sensor properly.

In another embodiment of the device, the physiological parameter sensors are selected from the group comprising body temperature sensors, pulse sensors, blood oxygen sensors, electromyographical sensors and/or electrocardiographical sensors. Hence, these sensor types may be present alone or as a combination of the aforementioned sensors. As already mentioned, physiological parameter sensors monitor the physical and physiological state of the person. This allows to better evaluate the well-being of the person conducting the exercises. For example, body temperature sensors may warn that a person has not performed a required warm-up prior to exercising. If the measured pulse is too high, the person may be overexerting himself. Likewise, blood oxygen sensors give information about the metabolical state. Electromyographical (EMG) sensors can supply information to determine whether a certain muscle is tired. Finally, electrocardiographical (ECG) sensors can also give early information whether the person is overexerting himself in an unsafe manner.

In an embodiment of the device, relative to a joint of the limb that the tube 2 is receiving, a sensor 4 is disposed proximal and another sensor 4 is disposed distal of the joint. This refers to the location of the sensors 4 on the tube 2 and therefore on the device 1 itself. For example, if the joint in question is the elbow joint, a motion sensor 4 placed proximal of the elbow joint would be on the upper arm. A motion sensor placed distal of the elbow joint would be on the lower arm. Likewise, if the joint in question is the knee joint, a motion sensor 4 placed proximal of the knee joint would be on the thigh and a motion sensor 4 placed distal of the knee joint would be on the lower leg. Having motion sensors on the proximal and distal side of a joint allows for monitoring the range of motion of the joint and thus generates motion data with a higher degree and quality of information.

In another embodiment of the device, the loop 3 is adapted to be worn around the neck or the waist of the person. For example, the diameter of the loop 3 may be in the range from ≥ 10 cm to ≤ 120 cm, preferably from ≥ 20 cm to ≤ 80 cm. Wearing the loop 3 around the neck covers the preferred way of securing a device 1 that is worn on the arm of the person. The device 1 is secured against slipping off the arm and against rotating on the arm. Likewise, wearing the loop 3 around the waist corresponds to the preferred way of securing a device 1 that is worn on the leg of the person.

In another embodiment of the device, the distal end of the tube 2 is adapted to receive a distal part of the limb within the tube 2 in a form-fitting manner. Simply put, a part of the distal end of the tube 2 stops a part of the limb inside the tube 2 from further moving outwards. In an equivalent consideration, this part of the limb stops the tube 2 from slipping proximally on the limb. This further fixes the position of the device and thus of the sensor or sensors 4 on the limb of the person. An example for this embodiment would be a hole near the distal end where a thumb or a toe could be put through.

Another example would be to close off the distal end of the tube 2 so a hand or a foot would abut the end in a sock-like manner. Yet another example would be to shape the distal end of the tube 2 in the form of a glove or a mitten to receive the hand of the person.

In another embodiment of the device, the tube 2 comprises a section where the stiffness of the material of the tube 2 is lower than the stiffness of the material of the remaining tube 2. Furthermore, this section is located on the tube 2 so as to be worn over a joint of the limb of the person. The stiffness is a measure for how much a material bends under its own weight. In the case of textiles, the stiffness may be determined according to ASTM D1388 "Standard Test Method for Stiffness of Fabrics". A tube 2 according to this embodiment will guide the person to wear it in a certain way. Because it is more comfortable to place the section of the tube 2 with the less stiff (in other words, softer) material on the inside of a joint, the person will place the tube on the limb accordingly. For example, when an elbow or a knee is flexed, the softer material on the inside of the joint may wrinkle easily, whereas the material on the outside of the joint is stiffer. In summary, this embodiment will help to secure a desired rotational orientation of the tube 2 and thus of the entire device 1 on the limb of the person. This in turn leads to a constant sensor orientation on the limb and is of particular benefit for muscle sensors such as EMG sensors. An advantageous combination of this embodiment together with outwardly extended sections to receive parts of the respective limb as already described above is also expressly contemplated in the present invention.

In another embodiment of the device, the sensor 4 is adapted to communicate wirelessly with another device. Such another device may be a data processing unit. Examples for wireless connections are the IEEE 802.11 protocols, the BLUETOOTH protocol or transmissions by infrared or visible light.

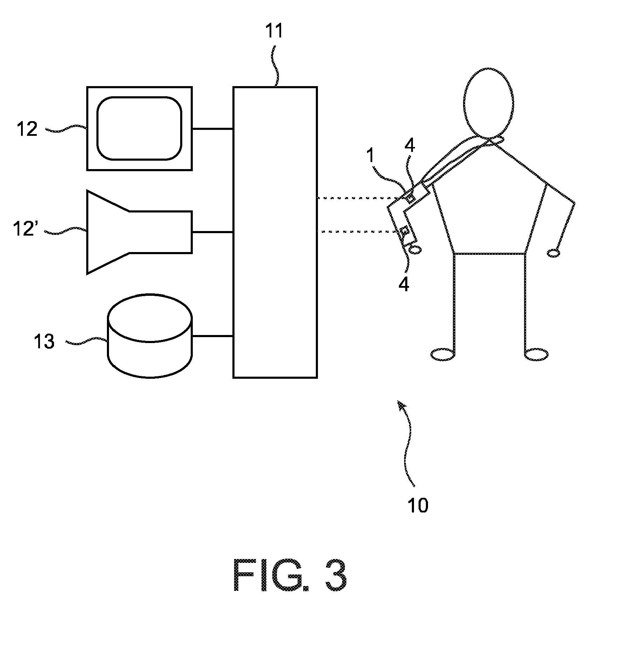

The system according to the present invention will now be described in general and with additional reference to FIG. 3. A system 10 for monitoring motion sequences of a person is shown which comprises a device 1 according to the invention and a data processing unit 11. The device 1 is worn by a person on his arm and with the loop around his neck. In the system 10, a sensor 4 of the device 1 is adapted to transmit sensor data to the data processing unit 11. This is depicted by the dashed lines between the two sensors 4 in question. Furthermore, the data processing unit 11 is adapted to perform calculations on the received sensor data. In particular, these calculations may be performed using a microprocessor in the data processing unit 11. Calculations to be performed on the sensor data include conversions of raw data into spatial coordinates of the sensors or deductions about the posture or movement of the person based on the sensor data.

In an embodiment of the system, the transmission between at least one sensor 4 of the device 1 and the data processing unit 11 is a wireless transmission. Examples for such transmissions are the IEEE 802.11 protocols, the BLUETOOTH protocol or transmissions by infrared or visible light. As shown in FIG. 3, the sensors 4 transmit their data to the processing unit 11.

In another embodiment of the system, it further comprises a feedback unit 12 in communication with the data processing unit 11. Such a feedback may be in the form of visual, acoustical or tactile feedback. A visual feedback unit may be a display that shows how the person moves and also shows an avatar performing the prescribed exercise movements. The display has the additional advantage that it may be used interactively, meaning that it can also function as an input device. An acoustical feedback unit may be a loudspeaker for giving spoken instructions to the person. Giving acoustical feedback has the advantage that the person is more independent in his movements as he does not have to watch a display. As shown in FIG. 3, a display 12 and a loudspeaker 12' may be included in the system 10.

In another embodiment of the system, the data processing unit 11 is adapted to perform calculations on the received sensor data in the form of comparing sensor data to stored data representing exercise motions. Stored data may be in the form of motion descriptions or motion templates. In simple cases, the stored data representing exercise motions may be an algorithm-based rule, such as the rule that in a particular exercise, the arm should not fall below a 90° angle. In the case of physiological parameter sensors being present in the system, the stored data may, for example, represent minimum or maximum muscle exertion, minimum or maximum pulse rates, and the like. Depending on the format of the stored data, the processing unit 11 will convert the sensor data into the appropriate format for comparison. The stored data may be located within the data processing unit or it may be on a separate storage medium. An external storage device 13 is shown in FIG. 3.

Whereas FIG. 3 has depicted the data processing unit 11 and attached peripherals as being separate from the device 1, it is also within the scope of the invention that the data processing unit and optionally other peripherals such as a feedback unit are located on the device 1 itself.

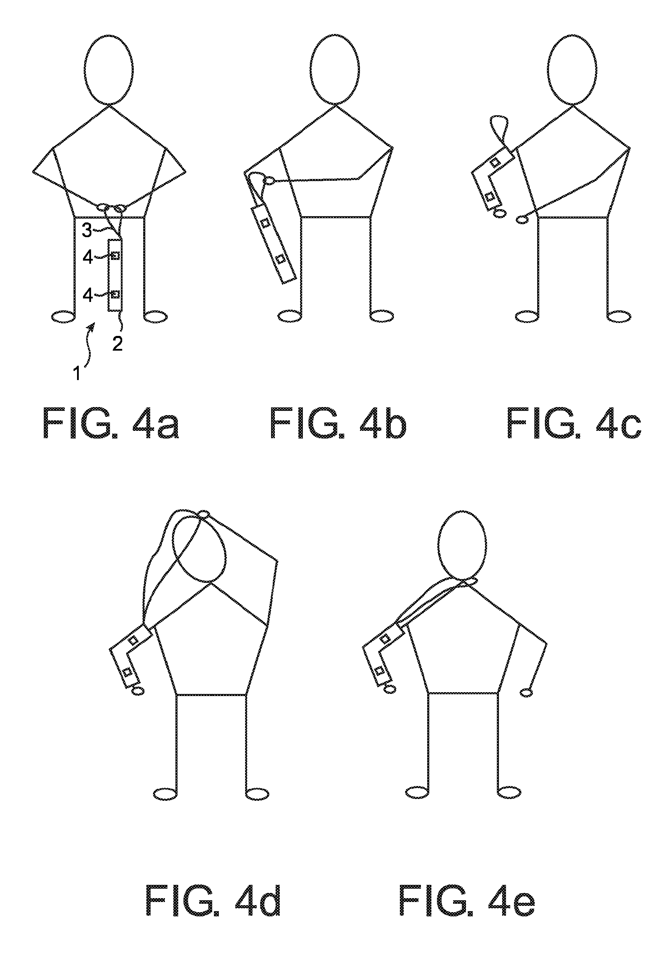

FIG. 4 shows how a device according to the invention is worn by showing a sequence of steps FIG. 4a to FIG. 4e. In the interest of clarity of the drawings, reference numerals have only been added to FIG. 4a. The numbering, of course, applies throughout FIG. 4. Schematically shown is a person who is suffering from a movement impairment of his right arm. It is on this right arm that the person wishes to wear the device 1 according to the invention. The starting position is shown in FIG. 4a where the person holds the device 1 in his hands. Then, in FIG. 4b, the person grasps the loop 3 with his left hand so as to aid the passing through of the impaired right arm through the tube 2 of device 1. After the device 1 has been fully pulled onto the arm as shown in FIG. 4c, the person pulls the loop 3 over his head in FIG. 4d. The last sequence step in FIG. 4e shows how the loop 3 is around the neck of the person, thereby securing the device 1 from slipping off. The person is now ready for exercising.

Turning now to the method according to the invention, in the first step a) a system 10 according to the invention is provided. This will include that the person wishing to perform exercise motions wears the device 1 according to the invention. Then the person starts exercising according to his schedule.

During the exercise the sensor or sensors 4 transmit their data to the data processing unit 11 of the system. This may be by a wired or a wireless connection as discussed above.

In step c) of the method the data is compared to stored data representing exercise motions. The comparison may be undertaken by the processing unit 11 of the system 10 as discussed above.

The results of the comparison may simply be stored fur future review, for example in order to assess the progress of the person in his exercises. Advantageously, in one embodiment the method further comprises the step of d) giving feedback to the person based on the result of the comparison in step c). The ways of giving feedback have already been outlined in connection with the description of the system. The decision to give feedback may, for example, depend on a pre-defined threshold for deviation of the exercise movement from exercise templates being violated.

It is to be noted that the system and the method according to the invention may also monitor exercise motions of a person even if only physiological parameter sensors are present. Exercise motions may be monitored, for example, with respect to muscle contractions. By this, neurological patients may train using a specific muscle instead of moving a limb with the help of other, unaffected muscles.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. The mere fact that certain features are recited in mutually different dependent claims does not indicate that a combination of these features cannot be used to an advantage.

The foregoing specification has illustrated the present invention with respect to physiotherapy exercises for a patient. However, the invention is by no means restricted to this. The present invention may, for instance, be used in the training of healthy persons wishing to learn a complex movement. An example for this is a golfer who would like to improve his golf swing.

Accordingly, the foregoing description is by way of example only and is not intended as limiting. The invention's scope is defined in the following claims and the equivalents thereto. Furthermore, reference signs used in the description and claims do not limit the scope of the invention as claimed.

## Claims

1. A device (1) for monitoring motion sequences of a person, comprising:
a flexible tube (2) with a proximal and a distal end, the tube being adapted to receive a limb of a person;
a loop (3) disposed at the proximal end of the tube (2), the loop (3) being adapted to be worn around a body part of the person; and
at least one sensor (4) connected to the tube (2), the sensor (4) being selected from the group comprising motion sensors and/or physiological parameter sensors.

2. The device according to claim 1, wherein the motion sensors are selected from the group comprising acceleration sensors, inertia sensors, gravity sensors, magnetometers, gyroscopes, optical tracking devices, a tilt meters, distance meters, position meters, goniometers and/or angle meters.

3. The device according to claim 1, wherein the physiological parameter sensors are selected from the group comprising body temperature sensors, pulse sensors, blood oxygen sensors, electromyographical sensors and/or electrocardiographical sensors.

4. The device according to claim 1, wherein relative to a joint of the limb that the tube (2) is receiving, a sensor (4) is disposed proximal and another sensor (4) is disposed distal of the oint.

5. The device according to claim 1, wherein the loop (3) is adapted to be worn around the neck or the waist of the person.

6. The device according to claim 1, wherein the distal end of the tube (2) is adapted to receive a distal part of the limb within the tube (2) in a form-fitting manner.

7. The device according to claim 1, wherein the tube 2 comprises a section where the elasticity of the material of the tube 2 is lower than the elasticity of the material of the remaining tube 2 and wherein this section is located on the tube 2 so as to be worn over a joint of the limb of the person.

8. The device according to claim 1, wherein the sensor (4) is adapted to communicate wirelessly with another device.

9. A system (10) for monitoring motion sequences of a person, comprising a device (1) according to claim 1 and a data processing unit (11), wherein a sensor (4) of the device (1) is adapted to transmit sensor data to the data processing unit (11) and the data processing unit (11) is adapted to perform calculations on the received sensor data.

10. The system according to claim 9, wherein the transmission between at least one sensor (4) of the device (1) and the data processing unit (11) is a wireless transmission.

11. The system according to claim 9, further comprising a feedback unit (12) in communication with the data processing unit (11).

12. The system according to claim 9, wherein the data processing unit (11) is adapted to perform calculations on the received sensor data in the form of comparing sensor data to stored data representing exercise motions.

13. A method for monitoring motion sequences of a person, comprising the steps of:
a) providing a system according to claim 9;
b) transmitting data from a sensor (4) to the data processing unit (11); and
c) comparing the data to stored data representing exercise motions.

14. The method according to claim 13, further comprising the step:
d) giving feedback to the person based on the result of the comparison in step c).

15. Use of a device (1) according to claim 1 for monitoring motion sequences of a person.
